# EUROPEAN PATENT SPECIFICATION

(11) **EP 0 473 698 B1**
(45) Date of publication and mention of the grant of the patent: **13.07.1994**
(21) Application number: 90908865.0
(22) Date of filing: 17.05.1990
(51) Int. Cl.: C07C 213/06, C07C 219/22

(54) **METHOD OF PREPARING D-PROPOXYPHENE**
VERFAHREN ZUR HERSTELLUNG VON D-PROPOXYPHEN
PROCEDE DE PREPARATION DE D-PROPOXYPHENE

(30) Priority: 26.05.1989 US 357564
(43) Date of publication of application: 11.03.1992
(73) Proprietor: MALLINCKRODT, INC., St. Louis, MO 63134 (US)
(72) Inventor: DUCHEK, John, R., St. Louis, MO 63125 (US)
(74) Representative: Eyles, Christopher Thomas
(86) International application number: US9002831
(87) International publication number: WO9014331

(56) References cited:
- US-A- 4 661 625
- JOURNAL OF THE AMERICAN CHEMICAL SOCIETY, vol. 77, no. 12, 28 June 1955, American Chemical Society, Washington, US; A. POHLAND et al, "Preparation of alpha-d- and alpha-l-4-dimethylamino-1,2-diphenyl-3-methyl-2-propionyloxybutane", pages 3400-3401

## Description

This invention relates to the preparation of d-propoxyphene, an analgesic, and its hydrochloride and napsylate salts.

### Background of the Invention

Of the many phenylpropylamines which show analgesic activity, the two most important are methadone and propoxyphene. The optically active alpha-dextro stereoisomer of propoxyphene is the only stereoisomer of propoxyphene which possesses analgesic properties. It is commonly used in its hydrochloride salt form which is a bitter, white crystalline powder freely soluble in water and soluble in alcohol. Its chemical name is α-d-1,2-diphenyl-2-propionoxy-3-methyl-4-dimethylaminobutane hydrochloride and is sold under several different trademarks including, for example, DARVON, DOLENE, and SK-65. The napsylate salt, i.e., the naphthalene sulfonate, is also used in many drug forms. It has previously been made from the hydrochloride salt.

Preparation of d-propoxyphene hydrochloride was first described by A. Pohland and H.R. Sullivan at J. Am. Chem. Soc., Volume 75, pp. 4458(1953). Therein, the authors disclosed a synthesis involving several stages, (1) preparation of an aminoketone called β-dimethylaminobutyrophenone by addition of the secondary amine, dimethylamine, to phenylpropenyl ketone; (2) a Grignard reaction of the amino ketone with benzylmagnesium chloride to yield the amino, hydrochloride-carbinols described as α-(75%) and β-(15%) 4-dimethylamino-1,2-diphenyl-3-methyl-2-butanol hydrochloride (sometimes hereinafter referred to as d-oxyphene hydrochloride); and (3) acylation of the α-amino carbinol hydrochloride by addition of an equal weight of propionic anhydride and five times that weight of pyridine and heating to reflux for several hours. Note the following reaction formula: 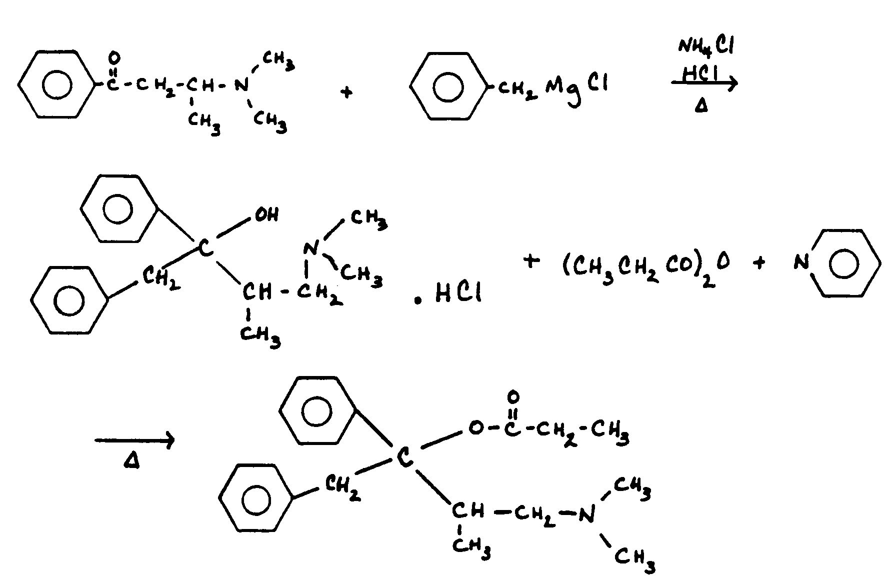
After cooling to recover the crude product, it was purified by two recrystallizations from methanol-ethyl acetate solution resulting in a yield of 70%.

Although this work confirmed that the α and not the β-diastereoisomers of propoxyphene gave rise to analgesic activity, it was still necessary to determine which of the optical forms of the α-diastereoisomer, i.e. α-d(+) or α-l(-), was responsible for the analgesic activity. Accordingly, Pohland and Sullivan reported in the J. Am. Chem. Soc., Volume 77, pp. 3400 (1955) their work on resolution of α-dl-4-dimethylamino-1, 2-diphenyl-3-methyl-2-butanol by fractional crystallization of its d-camphorsulfonic acid salt. From the respective α-d and α-l carbinol d-camphorsulfonic salts the optically active hydrochloride salts were prepared. The α-d-hydrochloride was acylated using propionic anhydride and triethylamine, while the α-l hydrochloride was acylated using propionic anhydride and pyridine. It was therein found that only the α-d stereoisomer gave the analgesic response. However, final purification of the hydrochloride salt required additional HCl and three recrystallizations and with yields of less than about 70%.

In 1963, Pohland, Peters and Sullivan reported in the J. Org. Chem., Vol. 28, pp. 2483, an alternative synthetic route for α-d-propoxyphene hydrochloride. Working backwards from the desired optically active isomer of propoxyphene by its hydrolysis and dehydration to stilbene, followed by ozonization of the stilbene, the authors discovered good yield of (-)-β-dimethylamino-α-methylpropiophenone. This optically active amino ketone was found to be surprisingly stable in salt form thus permitting its use as a starting material for a stereo selective synthesis of α-d-propoxyphene. Racemic β-dimethylamino-α-methylpropiophenone was resolved by crystallization of the dibenzoyl tartrate salts from acetone solution. The use of dibenzoyl-(-) -tartaric acid yielded the insoluble salt having (-)-β-dimethylamino-α-methylpropiophenone, while the use of the (+) tartaric acid yielded the salt having the (+) amino ketone isomer.

It is of interest that according to this reported synthesis, it was the (-) isomer of β-dimethylamino-α-methylpropiophenone, which when liberated from its (-) tartrate salt by Grignard reaction with benzylmagnesium chloride provided good yields of the (+) or (d) isomer α-1,2-diphenyl-3-methyl-4-dimethylamino-2-butanol which of course is the carbinol precursor for α-d-propoxyphene. The reported yields were 69%. The acylation was accomplished as had been previously reported, i.e., by means of propionic anhydride in either triethylamine or pyridine.

In 1978, Hungarian Pat. No. 14,441 disclosed a synthesis of α-d-propoxyphene employing the above-described method except that (1) the (+) tartaric acid was employed in the resolution of the racemic β-dimethylamino-α-methylpropiophenone and (2) the acylation was accomplished by reacting triethylamine in chloroform, propionyl chloride and the carbinol rather than propionyl anhydride and the carbinol hydrochloride. Still the product was precipitated in ether and required an amine catalyst.

Most recently, U.S. Patent Number 4,661,625 disclosed a synthetic method involving acylation of the carbinol (d-oxyphene) with propionyl chloride and thionyl chloride in dichloromethane. The yield of d-propoxyphene hydrochloride was improved to at least 76%, but use of the toxic additive thionyl chloride was required to get to that level. In addition, methylene chloride or another chlorinated solvent was required. Chlorinated impurities resulted and caused difficulties in purification.

However, a method that provides even higher yields of d-propoxyphene and its salts and doesn't require toxic and/or hazardous additives and solvents has long been highly desired. It is an object of the present invention to provide a means of producing d-propoxyphene in high yields without the need for amines or chlorinated solvents. It is a further object to provide methods of producing the hydrochloride and napsylate salts of d-propoxyphene in higher yields than previously obtainable.

### Summary of the Invention

D-propoxyphene is prepared by acylation of the free base carbinol, d-oxyphene, using propionic anhydride. Surprisingly, no additional solvent is needed. D-oxyphene is sufficiently soluble in propionic anhydride to react with it, but the reaction is less exothermic than expected so no additional solvent is required to dissipate the heat released and achieve sufficient temperature control to accomplish the acylation. Catalysts, preferably bases, may be used to increase the rate, but reaction times can be kept within acceptable limits without them. Surprisingly, water will increase the rate of reaction and may be present or deliberately added to the reaction mixture.

D-propoxyphene prepared by this method may be directly converted to the napsylate salt without previous conversion to the hydrochloride salt. This has the advantage of greatly increasing the efficiency of the preparation and the yield of the napsylate salt.

The present invention also provides a method of preparing the hydrochloride salt of d-propoxyphene free base using relatively inexpensive reactants. Higher overall yields result from the improved yields achieved in the acylation step.

### Detailed Description and Preferred Embodiments

D-oxyphene is well-known in the art and can be derived from any of several prior art methods, including those hereinbefore described, and is available commercially. In accordance with this invention, d-oxyphene may be reacted directly with propionic anhydride under acylating conditions requiring no amine or other additive and no chlorinated solvent. Although the reaction can proceed with equimolar amounts of d-oxyphene and the anhydride, it is preferred to use an excess of anhydride to act as the solvent and to drive the acylation reaction to completion. It is preferred to use at least 2.0 moles anhydride per mole of d-oxyphene. More preferably, a ratio of from 2.2 to 2.3 moles propionic anhydride per mole d-oxyphene is used. Less than an equimolar amount of propionic anhydride would yield d-propoxyphene, but the excess d-oxyphene will cause recovery and purification problems.

The solution of d-oxyphene in propionic anhydride is preferably heated in a nitrogen atmosphere. The temperature can range between about 50 and about 120 °C, preferably between 70 and 80 °C. Higher temperatures (above 90 °C) may promote formation of impurities.

The reaction time can vary according to the temperature employed, but will generally be between 2 and 10 hours, preferably 4 to 6 hours at the preferred temperatures.

Surprisingly, it has been found that water can increase the rate of the reaction. When present in a catalytic amount, about 0.2-0.4 moles per mole of d-oxyphene, a two-fold increase in the reaction rate has been observed. It is theorized that general base catalysis of the acylation reaction occurs before the water hydrolyzes propionic anhydride. Water may, therefore, be present during the reaction, and it may be preferable to add water to the reaction.

After the reaction is complete, the free base form of d-propoxyphene may be isolated in the usual manner. The preferred method is by precipitation from water or an ethanol-water mixture. Unreacted propionic anhydride is converted in water to propionic acid. This quenches the reaction. This acid, combined with the propionic acid produced by the acylation reaction, lowers the pH of the mixture and keeps the d-propoxyphene in solution. When the pH is raised to about 8.8 to 9.0, the free base will precipitate and can be recovered at greater than 95 percent yields. Ammonium hydroxide or other water soluble bases, such as sodium hydroxide or potassium hydroxide, can be used to raise the pH.

Purity of the recovered d-propoxyphene prepared by the present method is very good. It can be checked by nuclear magnetic resonance analysis or by liquid chromatography. It is highest when a mixture of ethanol and water is used to quench the propionic acid and anhydride reaction mixture. Ethanol helps keep any minor impurities in solution and produces the highest purity. This may occur due to formation of ethyl propionate which may have an increased solvent effect.

When ethanol it used, it should be washed from the recovered d-propoxyphene. This is preferably done by washing with deionized water, more preferably followed by reslurrying with additional washings in deionized water.

Once d-propoxyphene as the free base is recovered, it can be dried and stored for a time in this state. It can be converted to the hydrochloride salt or the napsylate salt before storage stability is of concern.

The present invention also includes the method of converting the free base to the hydrochloride salt. As the previous methods of preparation produced the hydrochloride salt without isolating the free base, an efficacious method of converting the free base to the hydrochloride salt had to be devised. It was found that the dried free base should be dissolved in ethyl acetate, which is then mixed with methanolic hydrogen chloride (methanol in which anhydrous HCl has been dissolved). It is preferred to use a molar equivalent of HCl. The hydrochloride salt precipitates from this mixture. Higher yields can be obtained by recycling the mother liquor, but good overall yields can be achieved without it due to the greatly improved yield in the acylation reaction.

The napsylate salt can be prepared from the hydrochloride salt by known methods. However, it has been found that the napsylate salt of d-propoxyphene can be prepared directly from the free base without completely drying it. After isolation, preferably by precipitation as described above, the free base solids are filtered to remove most of the water. Then the base is reslurried in water and one equivalent of hydrochloric acid is added. Sodium 2-naphthalene sulfonate (hereinafter called sodium napsylate) is added with ethanol. Preferably a slight molar excess of sodium napsylate is used, for example 1.1 moles per mole of free base. From this mixture d-propoxyphene napsylate may be crystallized. It is preferably washed and dried to the monohydrate state.

This procedure greatly improves the yield of the napsylate salt because the acylation step achieves high yields and losses inherent in the isolation of the hydrochloride salt are avoided.

The following examples are offered by way of illustration and are not limiting.

### Example 1

To a 5-litre flask equipped with an overhead stirrer, a nitrogen feed, thermometer, and heating mantle was added 2.0 kg (7.06 moles) d-oxyphene purchased from Merrell-Dow. To this was added 2.0 L (15.6 moles) propionic anhydride (Aldrich) with stirring and heating. The temperature was raised to 75-80 °C over 35 minutes and maintained at no more than 81 °C for four hours. The mixture was cooled to room temperature and then added dropwise to 10.0 L deionized water over 30 minutes. A clear yellow solution resulted. 1.85 L ammonium hydroxide was added to raise the pH to 8.8. Seed crystals of d-propoxyphene were added and white solids precipitated. The solution and precipitate were chilled by immersion in ice bath and filtered. The solid was dried by vacuum and then placed in a 60 °C oven for 2 days. The yield was 2390 g of white crystals, 99.7% of theory.

### Example 2

To a 22-litre vessel equipped with a stirrer, heating mantle, thermometer and a nitrogen feed was added 5.0 kg (17.6) moles d-oxyphene (Merrell-Dow). To it was added 5.0 L (39.0 moles) propionic anhydride (Eastman-Kodak). The temperature was raised and varied from 73-88 °C over 4 1/4 hours. The reaction mixture was split into two parts, each about 5L, and each was treated as follows: The mixture was slowly added to a mixture of 3.125 L absolute ethanol and 9.37 L deionized water. A mild odor of ethyl propionate was noted, but no phase separation was seen. The pH was adjusted to 8.8 with ammonium hydroxide, and white solids slowly precipitated. The mixture was chilled to approximately 9 °C and filtered with vacuum. The solids were washed twice on the filter with 2 L deionized water and reslurried in 10 L at room temperature. They were refiltered and again washed twice with 2 L deionized water. The solids were air-dried and analyzed by NMR, which showed no ethanol remaining. The combined yield was 6.12 kg, 102% of theory. m.p. 73.8 - 75.1 °C.

### Example 3

A 100-g sample of d-propoxyphene prepared as in Example 1 was dissolved in 481 mL ethyl acetate. 26.0 mL methanolic HCl (11.7 M) was added. The mixture was warmed to between 30 and 40 °C. The mixture then slowly crystallized. It was cooled to below 5 °C and filtered. The crystals were washed with 50 mL cold ethyl acetate. The yield was 79.3 g (72%).

Unconverted free base was recovered from the ethyl acetate filtrate by twice extracting with 100 mL of water acidified with 5 drops conc. HCl. The aqueous extracts were combined, and the remaining ethyl acetate was removed by blowing air over the solution.
When the ethyl acetate was completely removed, the pH was raised to 9.0 by adding ammonium hydroxide; solids formed that were filtered, washed and dried. 25.3 g d-propoxyphene were recovered. The total yield of salt and recovered free base was 94.5%.

### Example 4

A 40-g sample of d-propoxyphene prepared as in Example 2 was slurried in 169 mL deionized water with stirring and 10.9 mL conc. HCl were added.

Seventy mL ethanol were added; then 30.15 g sodium napsylate were added with stirring. The resulting slurry was heated to between 50 and 60 °C until a solution was obtained. The solution was filtered while hot and then allowed to cool, with stirring. Crystallization started on cooling. The solution was then chilled to less than 5 °C and filtered. The solids were washed with 210 mL deionized water and then reslurried in 195 mL deionized water. The slurry was stirred for 15 minutes, then filtered. The solids were again washed with 210 mL deionized water, collected, and dried overnight at 50-60 °C. The yield was 63.6 g (95.4 percent of theory).

## Claims

1. A method for preparing d-propoxyphene comprising
(a) dissolving d-oxyphene in propionic anhydride;
(b) reacting said d-oxyphene with said propionic anhydride under acylating conditions; and
(c) thereafter isolating d-propoxyphene.

2. A method according to Claim 1, wherein the amount of propionic anhydride is used in molar excess to the amount of d-oxyphene.

3. A method according to Claim 2, wherein the amount of propionic anhydride used is in a ratio of 2.2 to 2.3 moles per mole of d-oxyphene.

4. A method according to any one of Claims 1 to 3, wherein said reaction step is carried out under a nitrogen atmosphere.

5. A method according to any one of Claims 1 to 4, wherein said reaction step is carried out at temperatures from about 50 to about 120°C.

6. A method according to Claim 5, wherein said reaction step is carried out at temperatures from about 70 to about 80°C.

7. A method according to any one of Claims 1 to 6, wherein said isolation step is carried out by precipitation from water or an ethanol-water mixture having a pH of about 8.8 or above.

8. A method according to Claim 7, wherein said precipitation uses an ethanol-water mixture in a ratio ranging from 90 parts ethanol to 10 parts water to 10 parts ethanol to 90 parts water, on a volume basis.

9. A method according to Claim 7 or Claim 8, wherein said mixture of ethanol and water is in a ratio of about 50 parts ethanol to 50 parts water, on a volume basis.

10. A method according to Claim 7 or Claim 8, wherein said mixture of ethanol and water is in a ratio of about 25 parts ethanol to 75 parts water, on a volume basis.

11. A method according to any one of Claims 7 to 10, wherein said pH is achieved by addition of a sufficient amount of ammonium hydroxide, sodium hydroxide, or potassium hydroxide.

12. A method according to any one of Claims 1 to 11, additionally comprising adding a catalytic amount of water to said solution of d-oxyphene in propionic anhydride.

13. A method for preparing the hydrochloride salt of d-propoxyphene comprising
(a) preparing and isolating free base d-propoxyphene by a method according to any one of Claims 1 to 12;
(b) drying isolated d-propoxyphene to substantial dryness;
(c) dissolving said d-propoxyphene in ethyl acetate;
(d) adding methanolic hydrogen chloride; and
(e) isolating d-propoxyphene hydrochloride.

14. A method for preparing the napsylate salt of d-propoxyphene comprising
(a) preparing and isolating free base d-propoxyphene by a method according to any one of Claims 1 to 12;
(b) adding hydrochloric acid to said isolated d-propoxyphene;
(c) adding sodium napsylate; and
(d) isolating d-propoxyphene napsylate.

## Patentansprüche

1. Verfahren zur Herstellung von D-Propoxyphen, bei dem
(a) D-Oxyphen in Propionsäureanhydrid gelöst wird;
(b) das D-Oxyphen mit dem Propionsäureanhydrid unter acylierenden Bedingungen umgesetzt wird; und
(c) danach D-Propoxyphen isoliert wird.

2. Verfahren nach Anspruch 1, bei dem Propionsäureanhydrid in molarem Überschuß, bezogen auf die Menge an D-Oxyphen, eingesetzt wird.

3. Verfahren nach Anspruch 2, bei dem Propionsäureanhydrid in einer Menge von 2,2 bis 2,3 Mol je Mol D-Oxyphen eingesetzt wird.

4. Verfahren nach einem der Ansprüche 1 bis 3, bei dem die Umsetzungsstufe unter einer Stickstoffatmosphäre durchgeführt wird.

5. Verfahren nach einem der Ansprüche 1 bis 4, bei dem die Umsetzungsstufe bei Temperaturen von etwa 50 bis etwa 120°C durchgeführt wird.

6. Verfahren nach Anspruch 5, bei dem die Umsetzungsstufe bei Temperaturen von etwa 70 bis etwa 80°C durchgeführt wird.

7. Verfahren nach einem der Ansprüche 1 bis 6, bei dem die Stufe der Isolierung durch Ausfällen aus Wasser oder einem Ethanol-Wasser-Gemisch mit einem pH-Wert von etwa 8,8 oder darüber durchgeführt wird.

8. Verfahren nach Anspruch 7, bei dem zum Ausfällen ein Ethanol-Wasser-Gemisch in einem Verhältnis verwendet wird, das von 90 Teile Ethanol : 10 Teile Wasser bis 10 Teile Ethanol : 90 Teile Wasser, auf Volumenbasis, reicht.

9. Verfahren nach Anspruch 7 oder Anspruch 8, bei dem das Gemisch aus Ethanol und Wasser in einem Verhältnis von etwa 50 Teilen Ethanol und 50 Teilen Wasser, auf Volumenbasis, vorliegt.

10. Verfahren nach Anspruch 7 oder Anspruch 8, bei dem das Gemisch aus Ethanol und Wasser in einem Verhältnis von etwa 25 Teilen Ethanol und 75 Teilen Wasser, auf Volumenbasis, vorliegt.

11. Verfahren nach einem der Ansprüche 7 bis 10, bei dem der pH durch Zugabe einer ausreichenden Menge Ammoniumhydroxid, Natriumhydroxid oder Kaliumhydroxid eingestellt wird.

12. Verfahren nach einem der Ansprüche 1 bis 11, bei dem zusätzlich eine katalytische Menge Wasser zu der Lösung von D-Oxyphen in Propionsäureanhydrid zugegeben wird.

13. Verfahren zur Herstellung des Hydrochlorid-Salzes von D-Propoxyphen, bei dem
(a) D-Propoxyphen in Form der freien Base nach einem Verfahren gemäß einem der Ansprüche 1 bis 12 hergestellt und isoliert wird;
(b) das isolierte D-Propoxyphen im wesentlichen bis zur Trockne getrocknet wird;
(c) das D-Propoxyphen in Ethylacetat gelöst wird;
(d) methanolischer Chlorwasserstoff zugegeben wird; und
(e) D-Propoxyphen-Hydrochlorid isoliert wird.

14. Verfahren zur Herstellung des Napsilat-Salzes von D-Propoxyphen, bei dem
(a) D-Propoxyphen in Form der freien Base nach einem Verfahren gemäß einem der Ansprüche 1 bis 12 hergestellt und isoliert wird;
(b) Salzsäure zu dem isolierten D-Propoxyphen zugegeben wird;
(c) Natriumnapsilat zugegeben wird; und
(d) D-Propoxyphennapsilat isoliert wird.

## Revendications

1. Méthode pour la préparation du d-propoxyphène comprenant
(a) la dissolution du d-oxyphène dans l'anhydride propionique ;
(b) la réaction dudit d-oxyphène avec l'anhydride propionique dans des conditions acylantes ; et
(c) ensuite l'isolement du d-propoxyphène.

2. Méthode selon la revendication 1, dans laquelle on utilise une quantité d'anhydride propionique en excès molaire par rapport à la quantité de d-oxyphène.

3. Méthode selon la revendication 2, dans laquelle on utilise une quantité d'anhydride propionique dans un rapport situé entre 2,2 et 2,3 mole par mole de d-oxyphène.

4. Méthode selon une quelconque des revendications 1 à 3, dans laquelle ladite étape de réaction est effectuée sous une atmosphère d'azote.

5. Méthode selon une quelconque des revendications 1 à 4, dans laquelle ladite étape de réaction est effectuée à une température allant d'environ 50 à environ 120°C.

6. Méthode selon la revendication 5, dans laquelle ladite étape de réaction est effectuée à une température allant d'environ 70 à environ 80°C.

7. Méthode selon une quelconque des revendications 1 à 6, dans laquelle ladite étape d'isolement est effectuée par précipitation à partir d'eau ou d'un mélange éthanol-eau ayant un pH de 8,8 ou plus.

8. Méthode selon la revendication 7, dans laquelle ladite précipitation utilise un mélange éthanol-eau dont le rapport, exprimé en volumes, va de 90 parties d'éthanol pour 10 parties d'eau à 10 parties d'éthanol pour 90 parties d'eau.

9. Méthode selon la revendication 7 ou la revendication 8, dans laquelle ledit mélange éthanol-eau est dans un rapport, exprimé en volumes, d'environ 50 parties d'éthanol pour 50 parties d'eau.

10. Méthode selon la revendication 7 ou la revendication 8, dans laquelle ledit mélange éthanol-eau est dans un rapport, exprimé en volumes, d'environ 25 parties d'éthanol pour 75 parties d'eau.

11. Méthode selon une quelconque des revendications 7 à 10, dans laquelle ledit pH est obtenu par addition d'une quantité suffisante d'hydroxyde d'ammonium, d'hydroxyde de sodium ou d'hydroxyde de potassium.

12. Méthode selon une quelconque des revendications 1 à 11, comprenant en outre l'addition d'une quantité catalytique d'eau à ladite solution de d-oxyphène dans l'anhydride propionique.

13. Méthode pour la préparation du sel de l'acide chlorhydrique du d-propoxyphène comprenant
(a) la préparation et l'isolement de la base libre du d-propoxyphène par une méthode selon une quelconque des revendications 1 à 12 ;
(b) le séchage du d-propoxyphène isolé jusqu'à un degré de dessication substantiel ;
(c) la dissolution dudit d-propoxyphène dans l'acétate d'éthyle ;
(d) l'addition de chlorure d'hydrogène méthanolique ; et
(e) l'isolement du chlorhydrate de d-propoxyphène.

14. Méthode pour la préparation du sel de l'acide naphtalènesulfonique du d-propoxyphène comprenant
(a) la préparation et l'isolement de la base libre du d-propoxyphène par une méthode selon une quelconque des revendications 1 à 12 ;
(b) l'addition de chlorure d'hydrogène audit d-propoxyphène isolé ;
(c) l'addition de napsylate de sodium ; et
(d) l'isolement du napsylate de d-propoxyphène.
